# EUROPEAN PATENT APPLICATION

(11) **EP 4 613 299 A1**
(43) Date of publication of application: **10.09.2025**
(21) Application number: 25155503.3
(22) Date of filing: 03.02.2025
(51) Int. Cl.: A61M 5/168

(54) **GEL-COUPLED FORCE SENSOR TO DETECT BUBBLES AND BLOCKAGES IN FLUID USING LIGHT SOURCES**

(30) Priority: 08.03.2024 IN 202411016903
(71) Applicant: Honeywell International Inc., Charlotte, NC 28202 (US)
(72) Inventor: PADUBIDRI MURALI, HariPrasad, Charlotte, 28202 (US); HM, Manjunatha, Charlotte, 28202 (US); MAUT, Sandeep Laxman, Charlotte, 28202 (US)
(74) Representative: Haseltine Lake Kempner LLP

(57) **Abstract**

A device comprising a gel-coupled force sensor for detecting bubbles and/or blockages in fluids using light sources and/or ultrasonic transducers is provided. The devices may include a housing having a gel-coupled force sensor, wherein the gel-coupled force sensor comprises a substrate, at least one force sensing element, at least one application-specific integrated circuit (ASIC), a cover for the at least one force sensing element and the at least one ASIC, a gel for covering the force sensing element, a mesh layer proximate to the top of the gel, a barrier layer proximate to the mesh layer, and a membrane layer proximate to the barrier layer protruding at least a distance past an edge of the cover. The gel-coupled force sensor may surround a fluid flow tube. The housing may comprise a first light source and a second light source and/or an ultrasonic transducer.

## Description

### FIELD OF THE INVENTION

Embodiments of the present disclosure generally relate to devices comprising gel-coupled force sensors for detecting bubbles and blockages in fluids using light sources and ultrasonic transducers.

### BACKGROUND

There are many different devices for detecting bubbles and blockages in fluids. Some of these devices are used in the medical field, in some examples. For example, when a patient is receiving a fluid, an infusion pump may be equipped with bubble and/or blockage detection devices in order to detect bubbles and/or blockages, since the infusion of bubbles and/or blockages into the patient's bloodstream may result in adverse effects, including death, in some examples.

Applicant has identified many technical challenges and difficulties associated with such devices for detecting bubbles and/or blockages in fluids. Through applied effort, ingenuity, and innovation, many of these identified problems have been solved by developing solutions that are included in embodiments of the present disclosure, many examples of which are described in detail herein.

### BRIEF SUMMARY

Various example embodiments described herein relate to devices comprising gel-coupled force sensors for detecting bubbles and blockages in fluids using light sources and ultrasonic transducers.

In accordance with various embodiments of the present disclosure, a device is provided. In some embodiments, the device comprises a housing comprising a first light source and a second light source. In some embodiments, the device comprises a gel-coupled force sensor mounted in the housing such that the gel-coupled force sensor is proximate to a fluid flow tube for transporting a fluid and such that the housing surrounds the fluid flow tube. In some embodiments, the gel-coupled force sensor comprises: a substrate; at least one force sensing element coupled to the substrate; at least one application-specific integrated circuit (ASIC), wherein the ASIC is coupled to the substrate; and a gel for covering the at least one force sensing element, wherein the gel surrounds the force sensing element and wherein the gel is comprised within a cavity defined by a cover.

In some embodiments, the gel-coupled force sensor further comprises: a cover for the at least one force sensing element and the at least one ASIC, wherein the cover is coupled to the substrate; a mesh layer proximate to the gel, wherein the mesh layer is coupled to the cover; a barrier layer proximate to the mesh layer, wherein the barrier layer is coupled to the cover; and a membrane layer proximate to the barrier layer, wherein the membrane layer is coupled to the cover and wherein the membrane layer protrudes at least a distance past an edge of the cover.

In some embodiments, the substrate is comprised of a printed circuit board (PCB), and the PCB is comprised of a photosensitive material.

In some embodiments, the at least one force sensing element is coupled to the substrate via an adhesive or other binding agent, and the at least one force sensing element is wire-bonded to the substrate and to the at least one ASIC.

In some embodiments, the at least one ASIC is coupled to the substrate via an adhesive or other binding agent, and the at least one ASIC is wire-bonded to the substrate and to the at least one force sensing element.

In some embodiments, the gel is comprised of biocompatible gel.

In some embodiments, the mesh layer is comprised of at least one of: polyethylene or an acrylic-based material.

In some embodiments, the barrier layer is comprised of polyimide polymer.

In some embodiments, the membrane layer is comprised of silicone rubber.

In some embodiments, the first light source is proximate to the substrate and is configured to emit light through the fluid flow tube to illuminate the fluid contained within the fluid flow tube to measure a reference intensity, and the second light source is proximate to the fluid flow tube and is configured to emit light through the fluid flow tube to illuminate the fluid contained within the fluid flow tube to measure an actual intensity due to the occlusion.

In some embodiments, a center of an aperture of the second light source is configured to align with a center of the force sensing element, and a rigid body of the force sensing element is configured to provide a light path from the fluid contained within the fluid flow tube to the force sensing element.

In accordance with various embodiments of the present disclosure, a device is provided. In some embodiments, the device comprises an ultrasonic transducer. In some embodiments, the device comprises a gel-coupled force sensor mounted in the housing such that the gel-coupled force sensor is proximate to a fluid flow tube for transporting a fluid and such that the housing surrounds the fluid flow tube. In some embodiments, the gel-coupled force sensor comprises a substrate; at least one force sensing element coupled to the substrate; at least one application-specific integrated circuit (ASIC), wherein the ASIC is coupled to the substrate; and a gel for covering the at least one force sensing element, wherein the gel surrounds the force sensing element and wherein the gel is comprised within a cavity defined by a cover.

In some embodiments, the gel-coupled force sensor further comprises: a cover for the at least one force sensing element and the at least one ASIC, wherein the cover is coupled to the substrate; a mesh layer proximate to the top of the gel, wherein the mesh layer is coupled to the cover; a barrier layer proximate to the mesh layer, wherein the barrier layer is coupled to the cover; and a membrane layer proximate to the barrier layer, wherein the membrane layer is coupled to the cover and wherein the membrane layer protrudes at least a distance past an edge of the cover.

In some embodiments, the substrate is comprised of a printed circuit board (PCB).

In some embodiments, the at least one force sensing element is coupled to the substrate via an adhesive or other binding agent, and the at least one force sensing element is wire-bonded to the substrate and to the at least one ASIC.

In some embodiments, the at least one ASIC is coupled to the substrate via an adhesive or other binding agent, and the at least one ASIC is wire-bonded to the substrate and to the at least one force sensing element.

In some embodiments, the ultrasonic transducer comprises an emittance face configured to emit ultrasonic signals, wherein the emittance face is proximate to the fluid flow tube, and the force sensing element comprises a receiving face configured to receive ultrasonic signals for detecting a change in amplitude of the ultrasonic signals.

In accordance with various embodiments of the present disclosure, a gel-coupled force sensor is provided. In some embodiments, the gel-coupled force sensor comprises: a substrate; at least one force sensing element coupled to the substrate; at least one application-specific integrated circuit (ASIC), wherein the ASIC is coupled to the substrate; and a gel for covering the at least one force sensing element, wherein the gel surrounds the force sensing element and wherein the gel is comprised within a cavity defined by a cover.

In some embodiments, the gel-coupled force sensor further comprises: a cover for the at least one force sensing element and the at least one ASIC, wherein the cover is coupled to the substrate; a mesh layer proximate to the top of the gel, wherein the mesh layer is coupled to the cover; a barrier layer proximate to the mesh layer, wherein the barrier layer is coupled to the cover; and a membrane layer proximate to the barrier layer, wherein the membrane layer is coupled to the cover and wherein the membrane layer protrudes at least a distance past an edge of the cover.

In some embodiments, the at least one force sensing element is coupled to the substrate via an adhesive or other binding agent, and the at least one force sensing element is wire-bonded to the substrate and to the at least one ASIC; and the at least one ASIC is coupled to the substrate via an adhesive or other binding agent, and the at least one ASIC is wire-bonded to the substrate and to the at least one force sensing element.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a cross-sectional view of a gel-coupled force sensor, in accordance with some embodiments of the present disclosure;
FIG. 2 is a cross-sectional view of a device comprising a gel-coupled force sensor and two light sources;
FIG. 3 is a cross-sectional view of a device comprising a gel-coupled force sensor and an ultrasonic transducer;
FIG. 4 is an example flowchart illustrating an example of detecting bubbles and/or blockages in fluids using at least one light source; and
FIG. 5 is an example flowchart illustrating an example of detecting bubbles and/or blockages in fluids using at least one ultrasonic transducer, in accordance with some embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically coupled," "electrically coupling," "electrically couple," "electrically connected," "electrically connecting," "electrically connect," "in communication with," or "in electronic communication with" in the present disclosure refers to two or more elements or components being connected through wired means and/or wireless means, such that signals, electrical voltage/current, data and/or information may be transmitted to and/or received from these elements or components.

The term "in fluid communication with" in the present disclosure refers to two or more elements or components being connected through one or more paths or pathways, such that a fluid or other flowing media may be input to and/or output from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "sensor" refers to a component that may detect, measure, and/or identify any one or more attributes or characteristics of an environment or media, including but not limited to pressure(s).

In some examples, conventional fluid occlusion sensors are limited by various features. For example, a photosensitive force sensor may comprise a photosensitive force sensing element, two light sources, and a steel ball for transferring mechanical force due to occlusions present in the fluid. However, the steel ball may limit the amount of light the photosensitive force sensing element receives, in some examples, since the steel ball may block the light from the two light sources.

Embodiments of the present disclosure, in some examples, provide devices comprising gel-coupled force sensors for detecting bubbles and blockages in fluids using light sources and ultrasonic transducers.

Example embodiments of the devices described herein may include a housing having a gel-coupled force sensor, wherein the gel-coupled force sensor comprises a substrate, at least one force sensing element, at least one application-specific integrated circuit (ASIC), a cover for the at least one force sensing element and the at least one ASIC, a gel for covering the force sensing element, a mesh layer proximate to the top of the gel, a barrier layer proximate to the mesh layer, and a membrane layer proximate to the barrier layer protruding at least a distance past an edge of the cover. A housing for the gel-coupled force sensor may surround a fluid flow tube, for example, portions of the housing may surround the fluid flow tube such that the fluid flow tube is proximate to the gel-coupled force sensor. The housing may comprise a first light source and a second light source.

Alternatively or additionally, example embodiments of the devices described herein may include a housing having a gel-coupled force sensor, wherein the gel-coupled force sensor comprises a substrate, at least one force sensing element, at least one application-specific integrated circuit (ASIC), a cover for the at least one force sensing element and the at least one ASIC, a gel for covering the force sensing element, a mesh layer proximate to the top of the gel, a barrier layer proximate to the mesh layer, and a membrane layer proximate to the barrier layer protruding at least a distance past an edge of the cover. A housing for the gel-coupled force sensor may surround a fluid flow tube, for example, portions of the housing may surround the fluid flow tube such that the fluid flow tube is proximate to the gel-coupled force sensor. The housing may comprise an ultrasonic transducer.

Example embodiments of the present disclosure, in some examples, provide for the fluid flow tube being in direct mechanical contact with the membrane layer, such that the presence of an occlusion in the fluid would transmit pressure to the force sensing element. Example embodiments of the present disclosure provide for increased accuracy and/or precision in measuring the light from the light sources due to the absence of a steel ball, in some examples.

As described above, embodiments of the present disclosure, in some examples, provide devices for determining the presence and/or the size of occlusions present in fluids contained within fluid flow tubes, such as in infusion pumps in the medical setting.

To address challenges and limitations associated with devices for determining the presence of occlusions in fluids, various examples of the present disclosure may be provided. For example, various examples of the present disclosure may provide example devices for sensing the presence of occlusions in fluids, associated sensors, and associated methods. In some embodiments, the present disclosure may provide a device for sensing the presence of occlusions in fluids using one or more light sources and/or one or more ultrasonic transducers.

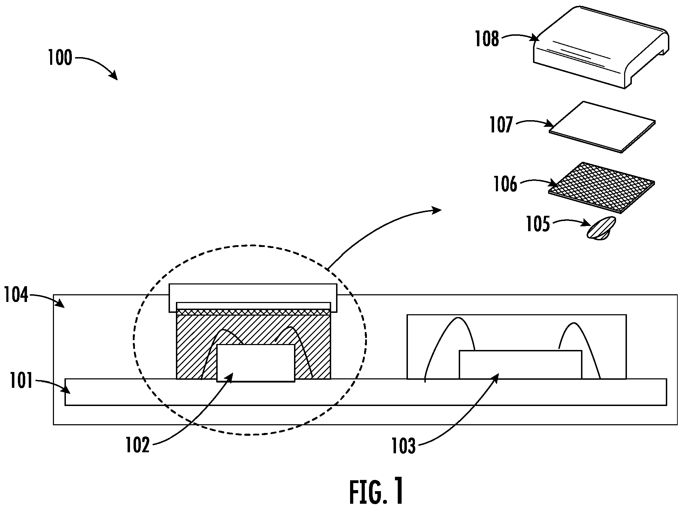

Referring now to FIG. 1, a cross-sectional view of a gel-coupled force sensor 100 is provided. The gel-coupled force sensor is mounted in a housing such that the gel-coupled force sensor is proximate to a fluid flow tube for transporting a fluid and such that the housing surrounds the fluid flow tube. The gel-coupled force sensor 100 comprises a substrate 101 for housing elements of the gel-coupled force sensor. The substrate 101 is comprised of printed circuit board (PCB), in some examples. The substrate 101 (e.g., the PCB) is comprised of photosensitive material, in some examples.

The substrate 101 comprises at least one force sensing element 102. In some examples, the at least one force sensing element 102 is used for sensing the force of an occlusion in a fluid, for example, in a fluid flow tube. The occlusion in the fluid flow tube may be a bubble, a blockage, and/or other occlusions. The force sensing element 102 comprises a rigid body configured to provide a light path from the fluid contained within the fluid flow tube to the force sensing element. The at least one force sensing element 102 is coupled to the substrate 101 via an adhesive and/or other binding agent.

The substrate 101 comprises at least one application-specific integrated circuit (ASIC) 103. The at least one ASIC 103 is coupled to the substrate 101 via an adhesive and/or other binding agent. The at least one force sensing element 102 is wire-bonded to the substrate 101, in some examples. The at least one ASIC 103 is wire-bonded to the substrate 101, in some examples. The at least one force sensing element 102 and the at least one ASIC may be wire-bonded to one another.

The gel-coupled force sensor 100 comprises a cover 104 for the at least one force sensing element 102 and/or the at least one ASIC 103. The cover 104 is mechanically coupled to the substrate 101, in some examples. For example, the cover 104 may snap onto or over the substrate. In some examples, the cover 104 may surround the substrate 101.

The gel-coupled force sensor 100 comprises a gel 105 for covering the at least one force sensing element. The gel 105 surrounds the at least one force sensing element 102. The gel 105 is comprised within the cavity around the at least one force sensing element 102 that is defined by or otherwise is created by the cover 104. In some examples, the gel 105 covers the wire-bonds which couple the at least one force sensing element 102 to the substrate 101. The gel 105 may be comprised of biocompatible gel and/or other materials.

The gel-coupled force sensor 100 comprises a mesh layer 106. The mesh layer 106 is proximate to I gel 105 (e.g., at the edge of the gel 105 opposite the side proximate to the substrate 101), in some examples. The mesh layer 106 may be comprised of polyethylene, an acrylic-based material, and/or other materials. In some examples, the mesh layer 106 is coupled to the cover 104. For example, the mesh layer 106 may be mechanically coupled to the cover 104.

The gel-coupled force sensor 100 comprises a barrier layer 107. The barrier layer 107 is proximate to the mesh layer 106 (e.g., the barrier layer 107 is laid on top of the mesh layer 106), in some examples. The barrier layer 107 may be comprised of polyimide polymer and/or other materials. In some examples, the barrier layer 107 is coupled to the cover 104. For example, the barrier layer 107 may be mechanically coupled to the cover 104.

The gel-coupled force sensor 100 comprises a membrane layer 108. The membrane layer 108 is proximate to the barrier layer 107 (e.g., the membrane layer 108 is laid on top of the barrier layer 107), in some examples. The membrane layer 108 protrudes at least a distance past or otherwise beyond an edge of the cover 104, in some examples. The membrane layer 108 may be comprised of silicone rubber and/or other materials. In some examples, the membrane layer 108 is coupled to the cover 104. For example, the membrane layer 108 may be mechanically coupled to the cover 104.

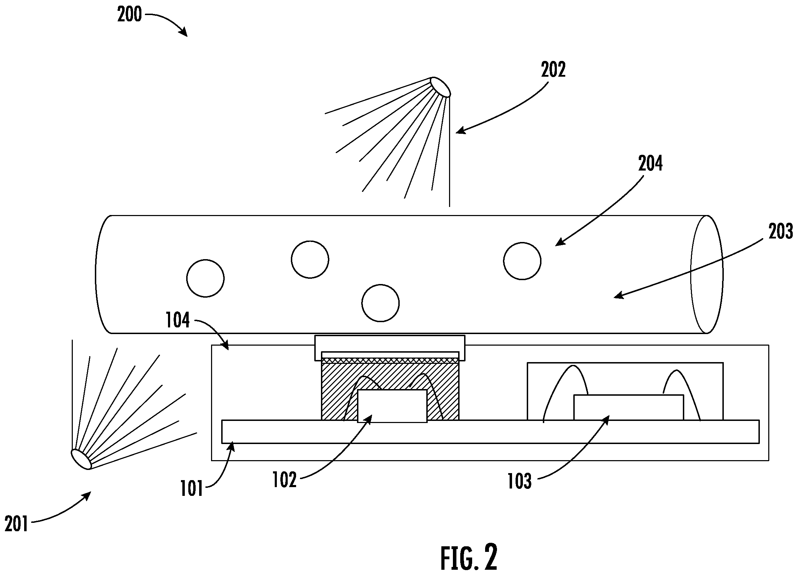

Referring now to FIG. 2, a cross-sectional view of a device 200 comprising a gel-coupled force sensor and two light sources is provided. The device 200 comprises a housing in which the gel-coupled force sensor 100 is mounted. The gel-coupled force sensor is mounted in the housing such that the gel-coupled force sensor is proximate to a fluid flow tube for transporting a fluid and such that the housing surrounds the fluid flow tube. The device 200 comprises the substrate 101 for housing elements of the gel-coupled force sensor. The substrate 101 is comprised of printed circuit board (PCB), in some examples. The substrate 101 (e.g., the PCB) is comprised of photosensitive material, in some examples.

The substrate 101 comprises the at least one force sensing element 102. In some examples, the at least one force sensing element 102 is used for and is otherwise configured for sensing the force of an occlusion in a fluid, for example, in a fluid flow tube. The occlusion in the fluid flow tube may be a bubble, a blockage, obstruction, and/or other occlusions. The force sensing element 102 comprises a rigid body configured to provide a light path from the fluid contained within the fluid flow tube to the force sensing element 102. The at least one force sensing element 102 is coupled to the substrate 101 via an adhesive and/or other binding agent.

The substrate 101 comprises the at least one ASIC 103. The at least one ASIC 103 is coupled to the substrate 101 via an adhesive and/or other binding agent. The at least one force sensing element 102 is wire-bonded to the substrate 101, in some examples. The at least one ASIC 103 is wire-bonded to the substrate 101, in some examples. The at least one force sensing element 102 and the at least one ASIC may be wire-bonded to one another.

The device 200 comprises the cover 104 for the at least one force sensing element 102 and/or the at least one ASIC 103. The cover 104 is mechanically coupled to the substrate 101, in some examples. For example, the cover 104 may snap onto or over the substrate. In some examples, the cover 104 may surround the substrate 101.

The device 200 comprises at least two light sources. The device 200 may comprise any number of light sources, which may increase accuracy in determining the presence of occlusions in fluids in a fluid flow tube. In some examples, the device 200 comprises a first light source 201 and a second light source 202. The first light source 201 is proximate to the substrate 101. The first light source 201 is configured to emit light through a fluid flow tube 203 to illuminate the fluid contained within the fluid flow tube 203 to measure a reference intensity. The second light source 202 is proximate to the fluid flow tube 203. In some examples, a center of an aperture of the second light source 202 is configured to align with a center of the force sensing element 102. In some examples, the rigid body of the force sensing element 102 is configured to provide alight path from the fluid contained within the fluid flow tube 203 to the force sensing element 102. The second light source 202 is configured to emit light through the fluid flow tube 203 to illuminate the fluid contained within the fluid flow tube to measure an actual intensity due to the occlusion.

The fluid flow tube 203 may be proximate to the membrane layer which is comprised in and/or surrounded by the gel-coupled force sensor. The fluid flow tube 203 may be positioned in between the second light source 202 and the cover 104. A housing for the gel-coupled force sensor 100 may surround a fluid flow tube, for example, portions of the housing may surround the fluid flow tube such that the fluid flow tube is proximate to the gel-coupled force sensor. The fluid flow tube 203 may comprise fluids such as medication, blood, and/or other fluids. The fluids within the fluid flow tube 203 may be one or more occlusions 204. The occlusions 204 may be comprised of one or more bubbles (e.g., pockets of air), one or more blockages, obstructions, and/or other occlusions 204.

The occlusions 204 may cause force to be imparted by the fluid flow tube onto the membrane 108, which transmits the force to the barrier layer 107, which transmits the force to the mesh layer 106, which transmits the force to the gel 105, which transmits the force to the force sensing element 102, which measures the force.

The actual intensity measured based on the second light source 202 is compared to the reference intensity measured based on the first light source 201 in order to determine whether an occlusion is present in the fluid, in some examples. For example, the reference intensity may indicate the intensity of the fluid when no occlusions are present. For example, the actual intensity may be lower than the reference intensity due to the presence of occlusions in the fluid.

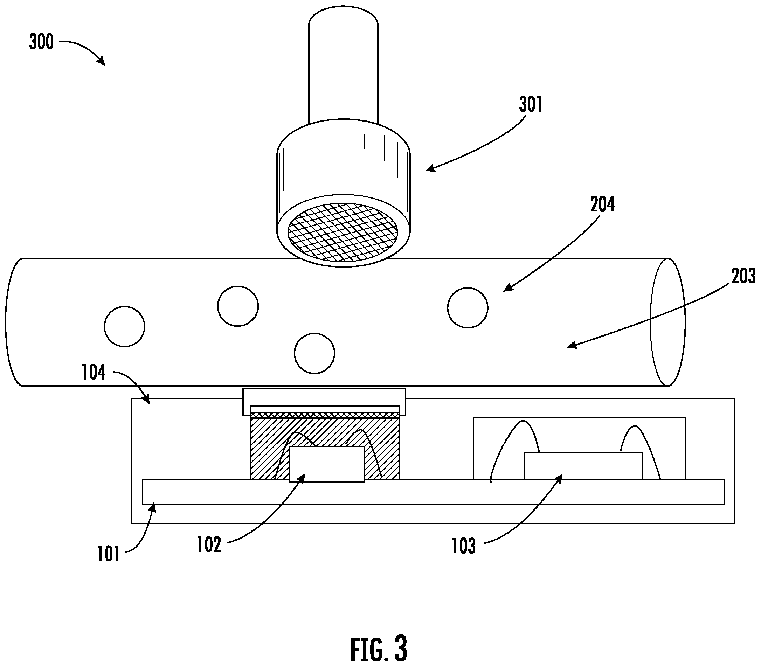

Referring now to FIG. 3, a cross-sectional view of a device 300 comprising a gel-coupled force sensor and an ultrasonic transducer is provided. The device 300 comprises a housing in which the gel-coupled force sensor 100 is mounted. The gel-coupled force sensor is mounted in the housing such that the gel-coupled force sensor is proximate to a fluid flow tube for transporting a fluid and such that the housing surrounds the fluid flow tube. The device 300 comprises the substrate 101 for housing elements of the gel-coupled force sensor. The substrate 101 is comprised of printed circuit board (PCB), in some examples. The substrate 101 (e.g., the PCB) is comprised of photosensitive material, in some examples.

The substrate 101 comprises the at least one force sensing element 102. In some examples, the at least one force sensing element 102 is used for sensing the force of an occlusion in a fluid, for example, in a fluid flow tube. The occlusion in the fluid flow 203 tube may be a bubble, a blockage, obstruction, and/or other occlusions. In some examples, the force sensing element 102 comprises a rigid body configured to provide a light path from the fluid contained within the fluid flow tube 203 to the force sensing element 102. The at least one force sensing element 102 is coupled to the substrate 101 via an adhesive and/or other binding agent.

The substrate 101 comprises the at least one ASIC 103. The at least one ASIC 103 is coupled to the substrate 101 via an adhesive and/or other binding agent. The at least one force sensing element 102 is wire-bonded to the substrate 101, in some examples. The at least one ASIC 103 is wire-bonded to the substrate 101, in some examples. The at least one force sensing element 102 and the at least one ASIC may be wire-bonded to one another.

The device 300 comprises the cover 104 for the at least one force sensing element 102 and/or the at least one ASIC 103. The cover 104 is mechanically coupled to the substrate 101, in some examples. For example, the cover 104 may snap onto or over the substrate. In some examples, the cover 104 may surround the substrate 101.

In some examples, device 300 comprises an ultrasonic transducer 301. The ultrasonic transducer 301 comprises an emittance face configured to emit ultrasonic signals. The emittance face of the ultrasonic transducer 301 is proximate to the fluid flow tube 203. In some examples, a center of the emittance face of the ultrasonic transducer is configured to align with a center of the force sensing element 102. The force sensing element 102 comprises a receiving face configured to receive ultrasonic signals for detecting a change in amplitude of the ultrasonic signals.

The fluid flow tube 203 may be proximate to the membrane layer which is comprised in and/or surrounded by the gel-coupled force sensor. The fluid flow tube 203 may be positioned in between the ultrasonic transducer 301 and the cover 104. The fluid flow tube 203 may comprise fluids such as medication, blood, and/or other fluids. The fluids within the fluid flow tube 203 may be one or more occlusions 204. The occlusions 204 may be comprised of one or more bubbles (e.g., pockets of air), one or more blockages, and/or other occlusions 204.

The occlusions 204 may cause force to be imparted by the fluid flow tube onto the membrane 108, which transmits the force to the barrier layer 107, which transmits the force to the mesh layer 106, which transmits the force to the gel 105, which transmits the force to the force sensing element 102, which measures the force.

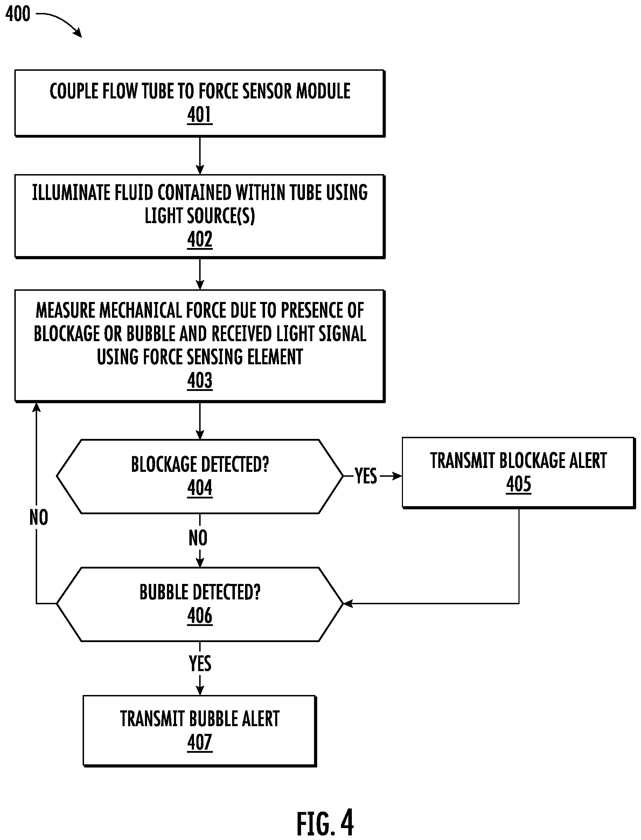

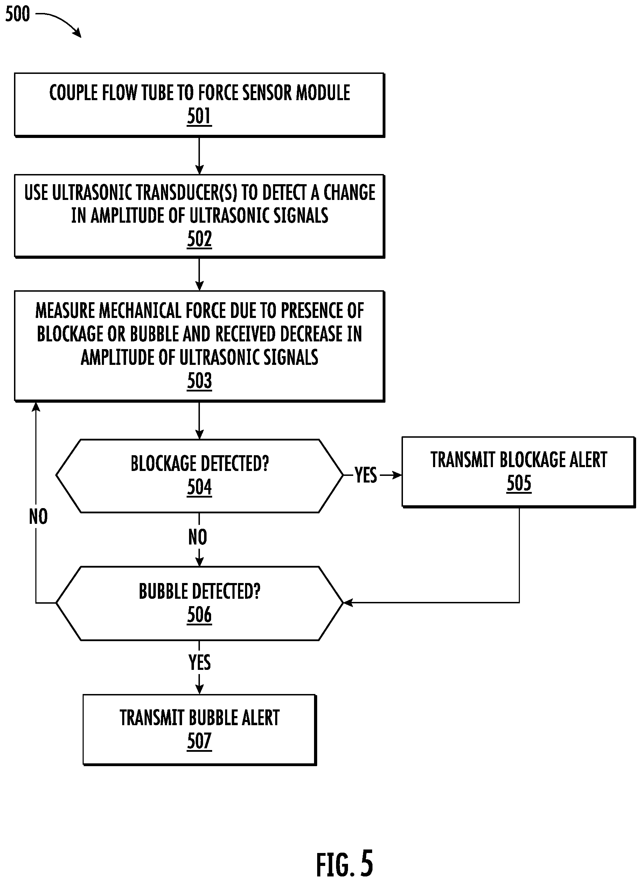

FIG. 4 and FIG. 5 are example flowcharts illustrating example methods of detecting occlusions in fluids using light sources and/or ultrasonic transducers. Referring now to FIG. 4, an example flowchart illustrating an example method 400 of detecting bubbles and/or blockages in fluids using at least one light source is provided.

At step/operation 401, a fluid flow tube is coupled to a force sensor. For example, the fluid flow tube 203 may be coupled to the gel-coupled force sensor 100.

At step/operation 402, a fluid contained within the fluid flow tube is illuminated using one or more light sources. For example, the light source 201 and/or the light source 202 may be used to illuminate the fluid contained within the fluid flow tube 203 in order to determine the presence of one or more occlusions in the fluid.

At step/operation 403, a force sensing element is used to measure the mechanical force due to the presence of an occlusion (e.g., one or more bubbles and/or blockages) and the received light signal from the one or more light sources, such as the first light source 201 and the second light source 202. For example, the force sensing element 102 may comprise a rigid body configured to provide a light path from the fluid contained within the fluid flow tube 203 to the force sensing element 102. The force due to the presence of a blockage may be measured by the force sensing element 102.

At step/operation 404, a determination is made as to whether a blockage is detected in the fluid. The actual intensity measured based on the second light source 202 is compared to the reference intensity measured based on the first light source 201 in order to determine whether a blockage is present in the fluid, in some examples. For example, the reference intensity may indicate the intensity of the fluid when no blockages are present. For example, the actual intensity may be lower than the reference intensity due to the presence of blockages in the fluid. The force due to the presence of a blockage may be measured by the force sensing element 102.

In the case of a "yes" determination at step/operation 404, at step/operation 405, a blockage alert is transmitted. The blockage alert may indicate that one or more blockages are present in the fluid contained within the fluid flow tube 203, in some examples. For example, the blockage may be caused by twisting in the fluid flow tube 203.

In the case of a "no" determination at step/operation 404, at step/operation 406, a determination is made as to whether a bubble is detected in the fluid. The actual intensity measured based on the second light source 202 is compared to the reference intensity measured based on the first light source 201 in order to determine whether a bubble is present in the fluid, in some examples. For example, the reference intensity may indicate the intensity of the fluid when no bubbles are present. For example, the actual intensity may be lower than the reference intensity due to the presence of bubbles in the fluid. The force due to the presence of a blockage may be measured by the force sensing element 102.

In the case of a "no" determination at step/operation 406, the method 400 returns to step/operation 403. In some examples, the force sensing element 102 is used to measure the mechanical force due to the presence of an occlusion (e.g., one or more bubbles and/or blockages) and the received light signal from the one or more light sources, such as the first light source 201 and the second light source 202. The force due to the presence of a blockage may be measured by the force sensing element 102.

In the case of a "yes" determination at step/operation 406, at step/operation 407, a bubble alert is transmitted. In some examples, the method 400 returns to step/operation 403. In some examples, the force sensing element 102 is used to measure the mechanical force due to the presence of an occlusion (e.g., one or more bubbles and/or blockages) and the received light signal from the one or more light sources, such as the first light source 201 and the second light source 202. The force due to the presence of a blockage may be measured by the force sensing element 102.

Referring now to FIG. 5, an example flowchart illustrating an example method 500 of detecting bubbles and/or blockages in fluids using at least one ultrasonic transducer is provided.

At step/operation 501, a fluid flow tube is coupled to a force sensor. For example, the fluid flow tube 203 may be coupled to the gel-coupled force sensor 100.

At step/operation 502, one or more ultrasonic transducers are used to detect a change in amplitude of ultrasonic signals. For example, the ultrasonic transducer 301, which comprises an emittance face configured to emit ultrasonic signals, emits ultrasonic signals through the fluid contained within the flow tube 203. In some examples, the force sensing element 102 comprises a receiving face configured to receive ultrasonic signals for detecting a change in amplitude of the ultrasonic signals. The ultrasonic signals may change in amplitude when they encounter an occlusion in the fluid within the fluid flow tube 203. The force due to the presence of a blockage may be measured by the force sensing element 102.

At step/operation 503, a force sensing element is used to measure the mechanical force due to the presence of an occlusion (e.g., one or more bubbles and/or blockages) and the decrease in amplitude of ultrasonic signals due to the presence of an occlusion. For example, the force sensing element 102, which comprises a receiving face configured to receive ultrasonic signals, may detect changes in amplitude of the ultrasonic signals due to the presence of an occlusion. The force due to the presence of a blockage may be measured by the force sensing element 102.

At step/operation 504, a determination is made as to whether a blockage is detected in the fluid. The amplitude of the emitted ultrasonic signals may decrease due to the presence of a blockage. The decreased amplitude may be measured by the receiving face of the force sensing element 102. The force due to the presence of a blockage may be measured by the force sensing element 102.

In the case of a "yes" determination at step/operation 504, at step/operation 505, a blockage alert is transmitted. The blockage alert may indicate that one or more blockages are present in the fluid contained within the fluid flow tube 203, in some examples. For example, the blockage may be caused by twisting in the fluid flow tube 203.

In the case of a "no" determination at step/operation 504, at step/operation 506, a determination is made as to whether a bubble is detected in the fluid. The amplitude of the emitted ultrasonic signals may decrease due to the presence of a blockage. The decreased amplitude may be measured by the receiving face of the force sensing element 102. The force due to the presence of a blockage may be measured by the force sensing element 102.

In the case of a "no" determination at step/operation 506, the method 500 returns to step/operation 503. In some examples, the force sensing element 102 is used to measure the mechanical force due to the presence of an occlusion (e.g., one or more bubbles and/or blockages) and the received decrease in amplitude of the ultrasonic signals from the ultrasonic transducer 301. The force due to the presence of a blockage may be measured by the force sensing element 102.

In the case of a "yes" determination at step/operation 506, at step/operation 507, a bubble alert is transmitted. In some examples, the method 500 returns to step/operation 403. In some examples, the force sensing element 102 is used to measure the mechanical force due to the presence of an occlusion (e.g., one or more bubbles and/or blockages) and the received decrease in amplitude of the ultrasonic signals from the ultrasonic transducer 301. The force due to the presence of a blockage may be measured by the force sensing element 102.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A device comprising:
a housing comprising a first light source and a second light source;
a gel-coupled force sensor mounted in the housing such that the gel-coupled force sensor is proximate to a fluid flow tube for transporting a fluid and such that the housing surrounds the fluid flow tube, the gel-coupled force sensor comprising:
a substrate;
at least one force sensing element coupled to the substrate;
at least one application-specific integrated circuit (ASIC), wherein the ASIC is coupled to the substrate; and
a gel for covering the at least one force sensing element, wherein the gel surrounds the force sensing element and wherein the gel is comprised within a cavity defined by a cover.

2. The device of claim 1, wherein the gel-coupled force sensor further comprises:
a cover for the at least one force sensing element and the at least one ASIC, wherein the cover is coupled to the substrate;
a mesh layer proximate to the gel, wherein the mesh layer is coupled to the cover;
a barrier layer proximate to the mesh layer, wherein the barrier layer is coupled to the cover; and
a membrane layer proximate to the barrier layer, wherein the membrane layer is coupled to the cover and wherein the membrane layer protrudes at least a distance past an edge of the cover.

3. The device of any of claims 1 or 2, wherein the substrate is comprised of a printed circuit board (PCB), and wherein the PCB is comprised of a photosensitive material.

4. The device of any of claims 1 to 3, wherein the at least one force sensing element is coupled to the substrate via an adhesive or other binding agent, and wherein the at least one force sensing element is wire-bonded to the substrate and to the at least one ASIC.

5. The device of any of claims 1 to 4, wherein the at least one ASIC is coupled to the substrate via an adhesive or other binding agent, and wherein the at least one ASIC is wire-bonded to the substrate and to the at least one force sensing element.

6. The device of any of claims 1 to 5, wherein the gel is comprised of biocompatible gel.

7. The device of any of claims 1 to 6, wherein the mesh layer is comprised of at least one of: polyethylene or an acrylic-based material.

8. The device of any of claims 1 to 7, wherein the barrier layer is comprised of polyimide polymer.

9. The device of any of claims 1 to 8, wherein the membrane layer is comprised of silicone rubber.

10. The device of any of claims 1 to 9, wherein the first light source is proximate to the substrate and is configured to emit light through the fluid flow tube to illuminate the fluid contained within the fluid flow tube to measure a reference intensity, and wherein the second light source is proximate to the fluid flow tube and is configured to emit light through the fluid flow tube to illuminate the fluid contained within the fluid flow tube to measure an actual intensity due to the occlusion.

11. The device of any of claims 1 to 10, wherein a center of an aperture of the second light source is configured to align with a center of the force sensing element, and wherein a rigid body of the force sensing element is configured to provide a light path from the fluid contained within the fluid flow tube to the force sensing element.

12. A system comprising:
an infusion pump; and
a blockage detection device for detecting blockages in fluids, the blockage detection device comprising:
a housing comprising a first light source and a second light source;
a gel-coupled force sensor mounted in the housing such that the gel-coupled force sensor is proximate to a fluid flow tube for transporting a fluid and such that the housing surrounds the fluid flow tube, the gel-coupled force sensor comprising:
a substrate;
at least one force sensing element coupled to the substrate;
at least one application-specific integrated circuit (ASIC), wherein the ASIC is coupled to the substrate; and
a gel for covering the at least one force sensing element, wherein the gel surrounds the force sensing element and wherein the gel is comprised within a cavity defined by a cover.

13. The system of claim 12, wherein the gel-coupled force sensor further comprises:
a cover for the at least one force sensing element and the at least one ASIC, wherein the cover is coupled to the substrate;
a mesh layer proximate to the gel, wherein the mesh layer is coupled to the cover;
a barrier layer proximate to the mesh layer, wherein the barrier layer is coupled to the cover; and
a membrane layer proximate to the barrier layer, wherein the membrane layer is coupled to the cover and wherein the membrane layer protrudes at least a distance past an edge of the cover.

14. The system of any of claims 12 or 13, wherein the first light source is proximate to the substrate and is configured to emit light through the fluid flow tube to illuminate the fluid contained within the fluid flow tube to measure a reference intensity, and wherein the second light source is proximate to the fluid flow tube and is configured to emit light through the fluid flow tube to illuminate the fluid contained within the fluid flow tube to measure an actual intensity due to the occlusion.

15. A method comprising:
mounting a gel-coupled force sensor in a housing such that the gel-coupled force sensor is proximate to a fluid flow tube for transporting a fluid and such that the housing surrounds the fluid flow tube, the gel-coupled force sensor comprising:
a substrate;
at least one force sensing element coupled to the substrate;
at least one application-specific integrated circuit (ASIC), wherein the ASIC is coupled to the substrate; and
a gel for covering the at least one force sensing element, wherein the gel surrounds the force sensing element and wherein the gel is comprised within a cavity defined by a cover;
coupling the cover to the substrate;
coupling a mesh layer to the cover;
disposing a barrier layer proximate to the mesh layer;
coupling the barrier layer to the cover;
disposing a membrane layer proximate to the barrier layer; and
coupling the membrane layer to the cover such that the membrane layer protrudes at least a distance past an edge of the cover.
